# EUROPEAN PATENT SPECIFICATION

(11) **EP 0 559 448 B1**
(45) Date of publication and mention of the grant of the patent: **15.07.1998**
(21) Application number: 93301612.3
(22) Date of filing: 03.03.1993
(51) Int. Cl.: G01T 1/161, G01T 1/26

(54) **Diamond radiation probe**
Diamant-Strahlungsdetektor
Détecteur de rayonnement à diamant

(30) Priority: 04.03.1992 ZA 921614
(43) Date of publication of application: 08.09.1993
(73) Proprietor: DE BEERS INDUSTRIAL DIAMOND DIVISION (PROPRIETARY) LIMITED, Johannesburg (ZA)
(72) Inventor: Nam, Tom Leong, Johannesburg, Transvaal (ZA); Keddy, Rex James, Johannesburg, Transvaal (ZA); Grobbelaar, Jacobus Hermanus, Alberton, Transvaal (ZA); Burns, Robert Charles, Johannesburg, Transvaal (ZA)
(74) Representative: Jones, Alan John

(56) References cited:
- EP-A- 0 314 458
- EP-A- 0 371 903
- PHYSICS IN MEDICINE AND BIOLOGY, vol. 32,no. 6, June 1987 pages 751-759, KEDDY,RJ ET AL 'synthetic diamonds as ionisation chamber radiation detectors in biological environments'
- PHYSICS IN MEDICINE AND BIOLOGY, vol. 26,no. 2, March 1981 BRISTOL, GB, pages 269-275, BURGEMEISTER, E A 'DOSIMETRY WITH A DIAMOND OPERATING AS A RESISTOR'

## Description

### BACKGROUND TO THE INVENTION

THIS invention relates to a diamond radiation probe.

Currently, dosimetric data for radiation therapy are usually obtained by means of field analysers which use ionisation chambers or silicon diodes as field and reference detectors. The former detector type suffers from poor spatial resolution, low speed of operation and perturbation effects, whilst the latter is disadvantaged by both energy and temperature dependent response characteristics.

The use of diamond as a conductive sensing element for the detection of ionising radiation or atomic emissions is well known, and is described in US Patents Nos. 4,833,328 and 5,012,108.

In an article in "PHYSICS IN MEDICINE & BIOLOGY", Vol 32, No. 6, June 1987, at pages 751 to 759, by Keddy R.J. et al. entitled "Synthetic Diamonds as Ionisation Chamber Radiation Detectors in Biological Environments", a diamond radiation probe is disclosed which is sensitive to ionising radiation. The probe comprises an elongate probe body having a front end terminating in a probe head at the front end thereof and a rear end. First and second electrodes are carried on the probe head, and mounting means in the form of non tissue-equivalent brass pincer jaws are provided for mounting a counting diamond between first and second non tissue-equivalent gold electrodes which are arranged to contact the opposite sides of the counting diamond. The gold electrodes extend laterally relative to the main axis of the probe. First and second electrically conductive leads extend rearwardly from the respective first and second electrodes, and are transversely spaced apart from one another.

EP-A-0 314 458 discloses a diamond radiation probe for measuring ionising radiation. The probe includes a probe body having a counting diamond to which a pair of electrical contacts is permanently attached. The contacts are metallic, or can be formed by using silver-loaded epoxy adhesive for attaching the conductors to ion implanted zones on the counting diamond. A pre-amplifier is connected directly to the contacts on the diamond for amplifying the pulses or conduction signal from the diamond.

Diamond sensing elements have yet to achieve widespread use in the routine monitoring of radiation fields. One of the principle reasons for this is that difficulties have been encountered in providing contacts for the diamond which are ohmic, tissue-equivalent and mechanically robust. The application of silverdag (silver-loaded epoxy) to, or carbon implantation and/or boron implantation into the diamond surfaces prior to the attachment of electrical leads do provide good ohmic contacts. However, such methods do not facilitate the fixture of mechanically robust leads to the diamond, and consequently limit the routine use of diamond as a reliable radiation detector.

If the material used to form the electrical contacts or surrounding the detector elements does not have an atomic number close to that of carbon, undesirable effects may be produced in that non-tissue-equivalent material is "seen" by the radiation as an artefact, as it lacks the same attenuation characteristics as soft tissue. This is especially true when accurate electron beam-profile monitoring is required.

### SUMMARY OF THE INVENTION

According to the invention, there is provided a diamond radiation probe which is sensitive to ionising radiations comprising:
a) an elongate probe body having a front end and a rear end and terminating in a probe head at the front end thereof;
b) first and second electrodes carried on the probe head;
c) mounting means for mounting a counting diamond between the first and second electrodes, which are arranged to contact opposite sides of the counting diamond; and
d) first and second electrically conductive leads extending rearwardly from the respective first and second electrodes at the front end of the probe body through the probe body towards the rear end thereof;
characterized in that the second electrode is disposed behind the first electrode, and in that the probe head is formed from a material which is near tissue-equivalent and the first and second electrodes are formed from a material which is near tissue-equivalent.

Preferably, a guard conductor is interposed between the first and second electrically conductive leads.

The term "near tissue-equivalent" means that the combination of elements in a material have an average atomic number from 7.1 to 7.6, and preferably 7.4. The predominant elements making up the combination may have atomic numbers ranging from 4 to 13. When subjected to ionising radiation, a material which is near tissue-equivalent would scatter the radiation in a manner similar to soft tissue, which has an average atomic number of 7.4.

Preferably, the first and second electrodes are disposed coaxially relative to a major axis of the probe.

Conveniently, the mounting means comprise clamping means for removably clamping the counting diamond within the head of the probe, the clamping means being arranged to move at least one of the electrodes axially relative to the other.

In an alternative form of the invention the mounting means comprises bonding means for bonding the first and second electrodes to the counting diamond.

The first electrically conductive lead is preferably an inner conductor which extends axially through the body of the probe, the second conductive lead includes an outer conductor which constitutes an outer shell of the probe body, and the guard conductor is an intermediate earth shield extending coaxially between the first and second conductive leads.

Electrical insulators are conveniently interposed between the earth shield and the first and second electrically conductive leads, the insulators being formed from a near tissue-equivalent material.

The bonding means is typically integral with the first and second electrodes, and is constituted by a bond in which the first and second leads are bonded or evaporated onto opposite sides of the counting diamond, by means of a carbon-loaded epoxy, for example.

The probe is advantageously arranged to be coupled to a triaxial cable having corresponding inner and outer conductors and an intermediate earth shield.

The first and second electrodes are typically arranged to sense incoming radiation both in the axial and the radial directions, relative to a major axis of the probe.

The first electrode is typically a ring-shaped electrode, and the second electrode is a pin-shaped electrode which is coaxial both with the ring-shaped electrode and with the major axis of the probe.

The head of the probe preferably has a maximum cross-sectional dimension of 10mm. The dimension is minimised to reduce the intrusive effect of the probe on the radiation field as is sensed by the counting diamond.

A liquid-proof sheath may be fitted over the probe, the sheath being formed from a material which is electrically insulating, tissue-equivalent and pervious to low energy electron and γ-radiation.

### BRIEF DESCRIPTION OF THE DRAWINGS

- **Figure 1**: shows a partly schematic side view of a first embodiment of a diamond radiation probe of the invention;
- **Figure 2**: shows a cross section on the line 2-2 of Figure 1;
- **Figures 3A to 3C**: show perspective views of alternative mounting arrangements of diamond material at the free end of the probe of Figures 1 and 2; and
- **Figure 4**: shows a partly exploded perspective view of an alternative embodiment of a diamond radiation probe of the invention.

### DESCRIPTION OF EMBODIMENTS

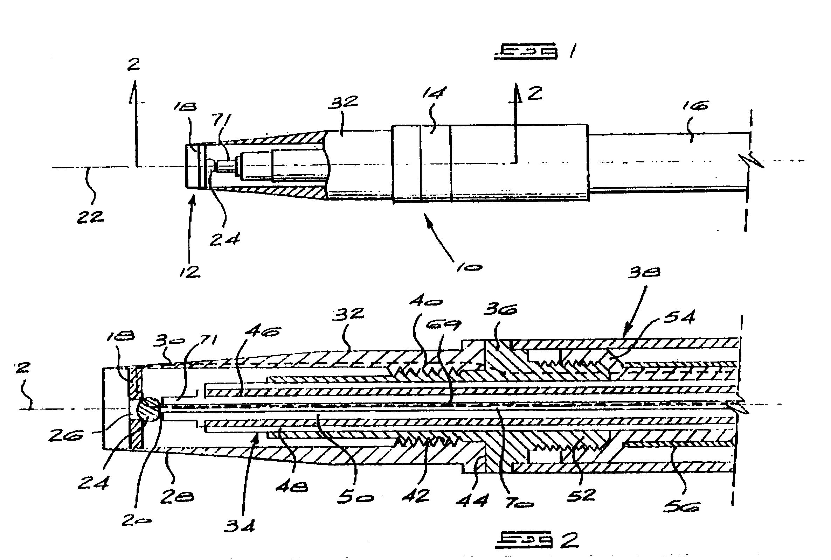

The diamond radiation probe illustrated in Figure 1 has a probe head 12 at its free end and an elongate probe body 14 at its fixed end which is mounted to a triaxial cable 16. The probe head 12 carries a first ring-shaped electrode 18 and a second pin-shaped electrode 20 which is aligned co-axially with the ring-shaped electrode 18 about a central major axis of symmetry 22 of the probe. A counting diamond 24 is clamped between the electrodes 18 and 20, with the inner edges of the aperture 26 of the ring-shaped electrode 18 serving to locate the diamond 24 along the central axis 22. The pin-shaped electrode 20 may optionally be spring-biased against the diamond 24 to hold it firmly in position against the edges of the circular aperture 26. The ring-shaped electrode 18 is formed integrally with a pair of arms 28 and 30 which extend axially from a tubular front housing 32. Both the ring-shaped electrode 18 and the front housing 32 are formed from a material having a low atomic number which is close to that of carbon, and is thus near tissue-equivalent, such as an epoxy resin. Other materials which could be used include aluminium, a conductive plastics material, or a conductive fibrous material such as carbon fibres embedded in a material which is near tissue-equivalent.

The pin-shaped electrode 20 forms the core of a central pillar portion 34 which is surrounded by the front housing 32. A central connection piece 36 connects the pillar portion 34 to a rear housing 38, which may be formed from an electrically conductive material such as aluminium. The central connection piece 36 has a tubular threaded spigot 40 onto which a complemental threaded socket 42 at the base of the front housing 32 is screwed. The base of the front housing 32 is spaced from a shoulder 44 on the connection piece when the diamond 24 is clamped in position.

A tubular earthed conductor 46 is spaced from the tubular spigot 40 and the central electrode pin 20 by means of respective tubular dielectric insulating members 48 and 50, which are formed from PTFE.

The connection piece 36 terminates in a rear threaded spigot 52, onto which a screening conductor 54 is mounted in a press fit. The screening conductor 54 is surrounded by a PTFE sleeve 56. The sleeve 56 is in turn surrounded by the rear tubular housing 38, which is mounted to and extends rearwardly from the connection piece 36. The base of the rear tubular housing 38 is arranged to accommodate the triaxial cable 16, by having a standard connection piece in order to enable the triaxial cable 16 to be mechanically fixed to it.

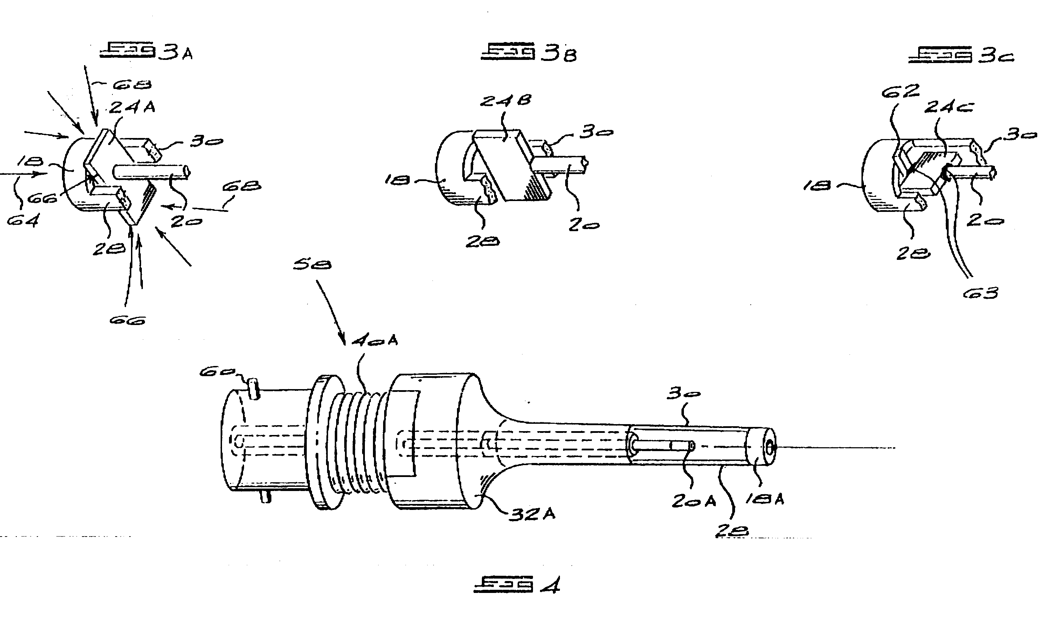

In Figure 4, an alternative embodiment of a radiation probe 58 is shown in which the fixed end of the probe is provided with a male bayonet connector 60 which is arranged to be coupled to a female bayonet connector at the end of a triaxial cable. In Figure 4, the front housing 32A of the probe is shown partly removed from the threaded spigot 40A. As the front housing 32A is screwed down over the threaded spigot 40A, the pin electrode 20A is moved closer to the ring electrode 18A. Clamping of the diamond 24 against the pin electrode 20 or 20A is thus effected by screwing the front housing 32 or 32A into position over the threaded spigot 40A.

In Figures 3A to 3C, alternative arrangements of mounting diamond sheets 24A, 24B and 24C are shown. The diamond sheets may be manufactured using a chemical vapour deposition technique, or may be cut from a diamond grown by high pressure or high temperature techniques. In Figure 3A, the major planar face of the diamond sheet 24A straddles the ring electrode 18, and the pin electrode 20 is brought against the opposite major face of the diamond sheet 24A in order to clamp it into position. In Figure 3B, the major edge of the diamond sheet 24B straddles the ring electrode 18, and the pin electrode 20 is brought up against the opposite major edge of the diamond sheet 24B in order to clamp it into position. In Figure 3C, a cross strut 62 straddles the ring electrode 18, and the major edge of the diamond sheet 24C rests in turn against the cross strut 62, which forms part of the ring electrode 18. The pin electrode 20 is clamped against the opposite major edge of the diamond sheet 24C. The diamond sheet 24C may also be bonded in position between the strut 62 and the pin electrode 20 using a carbon-loaded epoxy 63.

The ring electrode 18 allows for the monitoring of alpha-particle radiation travelling in an axial direction 64 through the front of the ring electrode. In addition, the relatively thin arms 28 and 30 provide windows 66 through which radiation emanating from the source alongside the probe in a radial direction 68 may also be monitored. This is particularly advantageous when the arms 28 and 30 are manufactured from a non-tissue equivalent material such as aluminium.

The triaxial cable 16 has an outer positive conductive sheath spaced from a central earthed conductor and an inner negative conductor. The positive, earth and negative conductors of the cable are connected to the respective positive conductive sheath 54, earth conductor 46 and central pin electrode 20 of the radiation probe. The current path, which is indicated in broken outline at 69 in Figure 2, extends from the positive sheath 54 to the connection piece 36, through the front housing 32 to the ring electrode 18, the counting diamond 24, and back along the pin electrode 20. The path 69 travels along a first conductive lead, which extends rearwardly from the first electrode 18, and which includes the front housing 32, the connection piece 36 and the positive sheath 54. The path similarly travels along a second conductive lead, which extends rearwardly from the pin electrode 20. In the embodiments of Figures 1 and 2, the second lead is constituted by a rear portion 70 of the pin electrode 20. The earthed conductor 46, acting as a guard, serves to prevent signal current leakage between the positive supply and high impedance sense conductors. In order to minimise the effects of air ionisation, the insulating PTFE sleeve 50 extends right up to the diamond 24, as is shown at 71. The front aluminium housing may also be coated either with polyethylene or PTFE.

In an alternative embodiment, the diamond element may be incorporated and enclosed within a housing which is formed from aluminium or another material having a low atomic number. The inside of the housing may be evacuated in order to reduce air ionisation effects. Alternatively, it may be filled with a near tissue-equivalent non-conducting material such as PTFE.

The entire probe 10 or 58 may be enveloped in a shrink wrap covering formed from material such as a rubber latex, which is electrically insulating, near tissue-equivalent and pervious to low energy electron and γ-radiation. The covering may be replaced each time the probe is inserted into a patient.

An advantage of the invention is that diamond elements with both prepared and unprepared surfaces may be mounted. For instance, diamond with implanted or laser scribed surfaces can easily be used, and diamonds having sizes varying from less than 1mm to a few millimetres, as well as diamond sheets fabricated using the chemical vapour deposition technique, can be utilised as radiation detectors. The probe electrodes 18 and 20 provide electrical contacts which are ohmic when clamped to a diamond which has had its surfaces treated by laser scribing or by implantation in the manner described in US Patent 4,833,328. For instance, boron implanted zones may be provided for enhancing ohmic contact between the diamond and the electrodes.

The electrical pathways provided within the probe are formed from materials such as aluminium, which are selected to have an atomic number which is as close to that of carbon as possible. The particular shape of the free end of the probe provides sufficient access for impinging radiation in both the axial and radial directions, and the probe is sufficiently robust to allow regular day-to-day handling without any ill effects. The probe may be constructed to whatever size is required in accordance with spatial resolution requirements. The combination of the triaxial cable and the coaxial positioning of the electrodes and the mounting means enables the maximum cross sectional dimension of the probe head to be typically reduced to 4mm. The position of the counting diamond within the probe head can thus be determined accurately, thereby increasing the spatial resolution of the probe and allowing it to measure radiation fields accurately.

The probe of the invention is designed to be compatible with conventional dosimetry equipment used, for instance, in various medical applications. For example, the probe can merely be plugged in as an input device in place of to an ionisation chamber without the need for extensive modifications in circuitry. The probe is particularly well suited for use in electron beam therapy using any electron therapy linear accelerator. However, it may also be used for γ- and X-radiations, ⁶⁰Co sources and medium and high voltage X-ray therapy generator units. In any of the above applications, the probe may be coupled to a type DI/4 electrometer system manufactured by Physikalisch-Techniche Werkstaetten AG of Freiburg, Germany, via a triaxial cable and a voltage matching network.

The probe may further be adapted for use with a PTW-MP3 water phantom system. Such a system comprises a tank containing water and means for moving a probe on a suitable mounting to various selected positions within the tank. At these positions the probe, which is fitted with a watertight jacket, detects radiation entering the tank from, for example, a linear accelerator, thereby allowing the radiation dosage to be measured at the various positions and collated by a computer.

The probe may also be used in various non-medical applications utilizing ionising radiation. For example, the probe may be used in radiation processing and/or sterilisation of foodstuffs, sewage water, and the radiation treatment of plastics, such as radiation polymerization for the crosslinking of polymers.

## Claims

1. A diamond radiation probe (10; 58) which is sensitive to ionising radiations comprising:
a) an elongate probe body (14) having a longitudinal axis and having a front end and a rear end and terminating in a probe head (12) at the front end thereof;
b) first and second electrodes (18, 20) carried on the probe head (12);
c) mounting means (32, 40, 40A, 42, 42A; 63) for mounting a counting diamond (24) between the first and second electrodes (18; 20), which are arranged to contact opposite sides of the counting diamond (24); and
d) first and second electrically conductive leads (32, 36, 54; 70) extending rearwardly from the respective first and second electrodes (18; 20) at the front end of the probe body (14) through the probe body towards the rear end thereof;
characterized in that the second electrode (20) is disposed axially behind the first electrode (18), and in that the probe head (12) is formed from a material which is near tissue-equivalent and the first and second electrodes are formed from a material which is near tissue-equivalent.

2. A diamond radiation probe according to claim 1 characterised in that a guard conductor (46) is interposed between the first and second electrically conductive leads (32, 36, 54; 70).

3. A diamond radiation probe according to claim 2 characterised in that the first electrically conductive lead is an inner conductor (70) which extends axially through the body (12) of the probe, the second electrically conductive lead includes an outer conductor which constitutes an outer shell (32) of the probe body, and the guard conductor (46) is an intermediate earth shield (46) extending coaxially between the first and second conductive leads.

4. A diamond radiation probe according to any one of the preceding claims characterised in that the first and second electrodes (18; 20) are disposed coaxially relative to a major axis (22) of the probe (14).

5. A diamond radiation probe according to any one of the preceding claims characterised in that the mounting means comprise clamping means (32, 40, 40A, 42, 42A) for removably clamping the counting diamond (24) within the head (12) of the probe, the clamping means being arranged to move at least one of the electrodes (20) axially relative to the other (18).

6. A diamond radiation probe according to any one of the preceding claims characterised in that the first and second electrodes (18; 20) are arranged to sense incoming radiation both in the axial and the radial directions, relative to a major axis (22) of the probe.

7. A diamond radiation probe according to claim 6 characterised in that the first electrode (18) comprises a ring-shaped electrode (18) and the second electrode is a pin-shaped electrode (20) which is coaxial both with the ring-shaped electrode and the major axis (22) of the probe.

8. A diamond radiation probe according to any one of the preceding claims characterised in that the probe head (12) has a maximum cross-sectional dimension of 10mm.

9. A diamond radiation probe according to any one of the preceding claims characterised in that electrical insulators (48; 50) are interposed between the earth shield (46) and the first and second electrically conductive leads (32, 36, 54; 70), the insulators being formed from a near tissue-equivalent material.

10. A diamond radiation probe according to any one of the preceding claims characterised in that the probe (10; 58) is arranged to be coupled to a triaxial cable (16) having corresponding inner and outer conductors and an intermediate earth shield.

11. A diamond radiation probe according to any one of the preceding claims characterised in that a liquid-proof sheath is fitted over the probe, the sheath being formed from a material which is electrically insulating, tissue-equivalent and pervious to low energy electron and γ-radiation.

## Patentansprüche

1. Diamantstrahlungssonde (10; 58), die auf ionisierende Strahlungen anspricht, mit:
a) einem länglichen Sondenkörper (14) mit einer Längsachse, einem vorderen Ende und einem hinteren Ende, der in einem Sondenkopf (12) am vorderen Ende endet;
b) an dem Sondenkopf (12) getragenen ersten und zweiten Elektroden (18, 20);
c) einer Befestigungseinrichtung (32, 40, 40A, 42A; 63) zum Anbringen eines Zähldiamanten (24) zwischen den ersten und zweiten Elektroden (18; 20), die derart angeodnet sind, daß sie mit gegenüberliegenden Seiten des Zähldiamanten (24) in Kontakt stehen; und
d) ersten und zweiten elektrisch leitenden Leitungen (32, 36, 54; 70), die sich jeweils von den ersten und zweiten Elektroden (18; 20) am vorderen Ende des Sondenkörpers (14) nach hinten durch den Sondenkörper zum hinteren Ende erstrecken;
dadurch gekennzeichnet, daß die zweite Elektrode (20) axial hinter der ersten Elektrode (18) angeordnet ist und daß der Sondenkopf (12) aus einem Material ausgebildet ist, das annähernd gewebeartig ist, und daß die ersten und zweiten Elektroden aus einem Material ausgebildet sind, das annähernd gewebeartig ist.

2. Diamantstrahlungssonde nach Anspruch 1, dadurch gekennzeichnet, daß zwischen den ersten und zweiten elektrisch leitenden Leitungen (32, 36, 54; 70) ein Schutzleiter (46) angeordnet ist.

3. Diamantstrahlungssonde nach Anspruch 2, dadurch gekennzeichnet, daß die erste elektrisch leitende Leitung ein innerer Leiter (70) ist, der sich axial durch den Körper (12) der Sonde erstreckt, die zweite elektrisch leitende Leitung ein äußerer Leiter ist, der eine äußere Hülle (32) des Sondenkörpers bildet, und der Schutzleiter (46) ein dazwischen vorgesehener Masseschirm (46) ist, der sich koaxial zwischen den ersten und zweiten leitenden Leitungen erstreckt.

4. Diamantstrahlungssonde nach einem der vorhergehenden Ansprüche, dadurch gekennzeichnet, daß die ersten und zweiten Elektroden (18; 20) koaxial relativ zu einer Hauptachse (22) der Sonde (14) angeordnet sind.

5. Diamantstrahlungssonde nach einem der vorhergehenden Ansprüche, dadurch gekennzeichnet, daß die Befestigungseinrichtung eine Klemmeinrichtung (32, 40, 40A, 42, 42A) zum entfernbaren Festklemmen des Zähldiamanten (24) innerhalb des Kopfes (12) der Sonde aufweist, wobei die Klemmeinrichtung derart angeordnet ist, daß sie wenigstens eine der Elektroden (20) relativ zu der anderen (18) axial bewegt.

6. Diamantstrahlungssonde nach einem der vorhergehenden Ansprüche, dadurch gekennzeichnet, daß die ersten und zweiten Elektroden (18; 20) derart angeordnet sind, daß sie einfallende Strahlung sowohl in axialer als auch in radialer Richtung relativ zu einer Hauptachse (22) der Sonde detektieren.

7. Diamantstrahlungssonde nach Anspruch 6, dadurch gekennzeichnet, daß die erste Elektrode (18) eine ringförmige Elektrode (18) aufweist und die zweite Elektrode eine stiftförmige Elektrode (20) ist, die sowohl mit der ringförmigen Elektrode als auch der Hauptachse (22) der Sonde koaxial ist.

8. Diamantstrahlungssonde nach einem der vorhergehenden Ansprüche, dadurch gekennzeichnet, daß der Sondenkopf (12) eine maximale Querschnittsabmessung von 10 mm aufweist.

9. Diamantstrahlungssonde nach einem der vorhergehenden Ansprüche, dadurch gekennzeichnet, daß zwischen dem Masseschirm (46) und den ersten und zweiten elektrisch leitenden Leitungen (32, 36, 54; 70) elektrische Isolatoren vorgesehen sind, wobei die Isolatoren aus einem annähernd gewebeartigen Material ausgebildet sind.

10. Diamantstrahlungssonde nach einem der vorhergehenden Ansprüche, dadurch gekennzeichnet, daß die Sonde (10; 58) mit einem Triaxialkabel (16) mit entsprechenden inneren und äußeren Leitern und einem dazwischen vorgesehenen Masseschirm verbindbar ist.

11. Diamantstrahlungssonde nach einem der vorhergehenden Ansprüche, dadurch gekennzeichnet, daß die Sonde mit einer flüssigkeitsdichten Hülle umhüllt ist, die aus einem Material ausgebildet ist, das elektrisch isolierend, gewebeartig und gegenüber Niedrigenergie-Elektronen- und γ-Strahlung durchlässig ist.

## Revendications

1. Sonde ou détecteur de rayonnement à diamant (10; 58) sensible aux radiations ionisantes, comprenant :
a) un corps de sonde oblong (14) ayant un axe longitudinal et une extrémité avant et une extrémité arrière et se terminant dans une tête de sonde (12) sur son extrémité avant ;
b) des première et seconde électrodes (18, 20) montées sur la tête de sonde (12) ;
c) des moyens de montage (32, 40, 40A, 42, 42A ; 63) destinés à monter un diamant de comptage (24) entre les première et seconde électrodes (18 ; 20) qui sont disposées pour venir en contact avec les côtés opposés du diamant de comptage (24) ; et
d) des premier et second fils conducteurs électriquement (32, 36, 54 ; 70) s'étendant vers l'arrière à partir des première et seconde électrodes respectives (18 ; 20) sur l'extrémité avant du corps de sonde (14) à travers le corps de sonde vers son extrémité arrière ;
caractérisé en ce que la seconde électrode (20) est disposée axialement derrière la première électrode (18) et en ce que la tête de sonde (12) est constituée d'un matériau qui est quasiment équivalent au tissu et les première et seconde électrodes sont réalisées à partir d'un matériau qui est pratiquement équivalent au tissu.

2. Sonde ou détecteur de rayonnement à diamant selon la revendication 1, caractérisé en ce qu'un conducteur de garde (46) est intercalé entre les premier et second fils conducteurs électriquement (32, 36, 54 ; 70).

3. Sonde ou détecteur de rayonnement à diamant selon la revendication 2, caractérisé en ce que le premier fil conducteur électriquement est un conducteur interne (70) qui s'étend axialement à travers le corps (12) du détecteur, le second fil conducteur électriquement comprend un conducteur extérieur qui constitue une enveloppe extérieure (32) du corps de sonde et le conducteur de garde (46) est une protection de terre intermédiaire (46) s'étendant coaxialement entre les premier et second fils conducteurs.

4. Sonde ou détecteur de rayonnement à diamant selon l'une quelconque des revendications précédentes, caractérisé en ce que les première et seconde électrodes (18 ; 20) sont disposées coaxialement par rapport à un grand axe (22) de la sonde (14).

5. Sonde ou détecteur de rayonnement à diamant selon l'une quelconque des revendications précédentes, caractérisé en ce que les moyens de montage comprennent des moyens de bridage (32, 40, 40A, 42, 42A) destinés à brider de façon amovible le diamant de comptage (24) à l'intérieur de la tête (12) de la sonde, les moyens de bridage étant aptes à déplacer au moins l'une des électrodes (20) axialement par rapport à l'autre (18).

6. Sonde ou détecteur de rayonnement à diamant selon l'une quelconque des revendications précédentes, caractérisé en ce que les première et seconde électrodes (18 ; 20) sont aptes à capter l'arrivée de rayonnement à la fois dans les directions axiale et radiale par rapport à un grand axe (22) de la sonde.

7. Sonde ou détecteur de rayonnement à diamant selon la revendication 6, caractérisé en ce que la première électrode (18) comprend une électrode annulaire (18) et la seconde électrode est une électrode en forme d'épingle (20) qui est coaxiale à la fois par rapport à l'électrode annulaire et au grand axe (22) de la sonde.

8. Sonde ou détecteur de rayonnement à diamant selon l'une quelconque des revendications précédentes, caractérisé en ce que la tête de sonde (12) présente une dimension en coupe transversale maximum de 10 mm.

9. Sonde ou détecteur de rayonnement à diamant selon l'une quelconque des revendications précédentes, caractérisé en ce que des isolateurs électriques (48 ; 50) sont intercalés entre la protection de terre (46) et les premier et second fils conducteurs électriquement (32, 36, 54 ; 70), les isolateurs étant réalisés à partir d'un matériau pratiquement équivalent au tissu.

10. Sonde ou détecteur de rayonnement à diamant selon l'une quelconque des revendications précédentes, caractérisé en ce que la sonde (10 ; 58) peut être raccordée à un câble triaxial (16) comportant des conducteurs intérieur et extérieur correspondants et une protection de terre intermédiaire.

11. Sonde ou détecteur de rayonnement à diamant selon l'une quelconque des revendications précédentes, caractérisé en ce qu'une enveloppe étanche au liquide est fixée sur la sonde, l'enveloppe étant réalisée à partir d'un matériau qui est électriquement isolant, équivalent au tissu et perméable au rayonnement γ et électron basse énergie.
